(19) Europäisches Patentamt
European Patent Office
Office européen des brevets

(11) **EP 3 705 019 A1**

(12) **EUROPEAN PATENT APPLICATION**

(43) Date of publication:
**09.09.2020  Bulletin 2020/37**

(51) Int Cl.:
**A61B 1/00** *(2006.01)*  **A61B 1/005** *(2006.01)*
**A61M 25/00** *(2006.01)*  **A61M 25/01** *(2006.01)*

(21) Application number: **19160528.6**

(22) Date of filing: **04.03.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Ambu A/S**
**2750 Ballerup (DK)**

(72) Inventors:
- **MATTHISON-HANSEN, Kaspar Mat**
  **3000 Helsingør (DK)**
- **HANSEN, Jan Guldberg**
  **2670 Greve (DK)**
- **JENSEN, Thomas Bachgaard**
  **1721 Copenhagen V (DK)**

(74) Representative: **AWA Denmark A/S**
**Strandgade 56**
**1401 Copenhagen K (DK)**

(54) **TIP PART FOR AN ENDOSCOPE**

(57)    A bendable articulated tip part for an endoscope, comprising a bending section having a number of hingedly connected segments including a proximal end segment, a distal end segment, and a plurality of intermediate segments positioned between the proximal end segment and the distal end segment,
wherein at least one segment chosen from the group consisting of the proximal end segment, the distal end segment, and the plurality of intermediate segments includes a surface having an area profile with a two-dimensional, arithmetical mean height parameter, $S_a$, of at least 0.9 micrometres as measured by the standard ISO 25178.

Fig. 2

EP 3 705 019 A1

**Description**

The present disclosure relates to endoscopes, and more specifically to an articulated tip part for an endoscope.

Endoscopes are well known for visually inspecting inaccessible parts of a natural or artificial body, such as human body cavities. Typically, the endoscope comprises an elongated insertion tube with a handle at the proximal end, as seen from the operator, and visual inspection means, such as a built-in camera assembly, at the distal end of the elongated insertion tube. This definition of the terms distal and proximal, i.e. proximal being the end closest to the operator and distal being the end remote from the operator, as used herein for endoscopes in general, is adhered to in the present disclosure.

As the name indicates, endoscopes are used for seeing inside things, such as lungs or other human body cavities of a patient. Modern endoscopes are therefore typically equipped with a light source and a vision receptor including a vision sensor, such as a camera or an image sensor. Provided that sufficient light is present, it is possible for the operator to see where the endoscope is steered and to set the target of interest once the tip has been advanced thereto. This therefore normally requires illumination of the area in front of the distal tip of the endoscope, in particular the field of vision of the camera(s). The light source, such as a light emitting diode or an optical fibre, may provide illumination.

Electrical wiring for the camera and other electronics, such as LED lighting accommodated in the tip part at the distal end, run along the inside of the elongated insertion tube from the handle to the tip part. Instead of using cameras, endoscopes may also be fibre-optic, in which case the optical fibres run along the inside of the elongated insertion tube to the tip part. For some applications, a working or suction channel may run along the inside of the insertion tube from the handle to the tip part, e.g. allowing liquid to be removed from the body cavity or allowing for insertion of surgical instruments or the like, into the body cavity. The suction channel may be connected to a suction connector, typically positioned at a handle at the proximal end of the insertion tube. For other applications, the working or suction channel may be omitted. A tubular sleeve typically covers the tip part. The tubular sleeve may be positioned around the tip part by applying a pressure differential to the tubular sleeve to increase the inner circumference of the tubular sleeve, then inserting the tip part into the tubular sleeve and relaxing the pressure differential so that the tubular sleeve wraps around the tip part.

In order to be able to manoeuvre the endoscope inside the body cavity, the distal end of the endoscope may comprise a bending section with increased flexibility, e.g. an articulated tip part allowing the operator to bend this section. Typically this is done by tensioning or slacking steering wires also running along the inside of the elongated insertion tube from the articulated tip part to a control mechanism of the handle.

The tip part of the present disclosure is intended for use in a single-use or disposable endoscope, it is therefore desired to reduce cost including manufacturing cost.

The preferred way of assembling the parts of the tip part is by adhesion as this is low-cost, improves liquid tightness, and flexible as adhesion can typically be implemented with a wide range of part geometries. The bending section is generally made of an elastic polyolefin material to allow sufficient elasticity for a high quality manoeuvering of the endoscope, while still keeping material costs low to allow the manufacture of a single-use endoscope. However, a shortcoming of this group of materials is that they are usually difficult to adhere to, and thus presents a challenge when assembling the parts of the endoscope.

It is therefore desirable to provide an improved tip part, and a method for obtaining the tip part, capable of eliminating or mitigating the shortcomings of prior tip parts and methods of obtaining the same.

On this background, it may be seen as an object of the present disclosure to provide an improved articulated tip part for an endoscope.

A first aspect of the present disclosure relates to a bendable articulated tip part for an endoscope, comprising a bending section having a number of hingedly connected segments including a proximal end segment, a distal end segment, and a plurality of intermediate segments positioned between the proximal end segment and the distal end segment,

wherein at least one segment chosen from the group consisting of the proximal end segment, the distal end segment, and the plurality of intermediate segments includes a surface having an area profile with a two-dimensional, arithmetical mean height parameter, $S_a$, of at least 0.9 micrometres as measured by the standard ISO 25178.

Experiments have shown that by providing a tip part according to the first aspect of the present disclosure, a number of advantages may be achieved.

An advantage may be that further assembling the bending section with a tubular sleeve for covering at least the intermediate segments may be further facilitated in that surprisingly the tubular sleeve may more easily be slid over the segments of the bending section. A typical way of assembling the tubular sleeve in the prior art is to apply a pressure differential, such as vacuum or an air blast, so as to increase the circumference of the tubular sleeve and thereby allow insertion of the bending section into the tubular sleeve, and thereafter reduce the pressure differential until the tubular sleeve wraps around the bending section to cover the intermediate segments. However, in a tip part according to the first aspect of the present disclosure, a lower pressure differential may be utilized or the tubular sleeve may even simply be manually slid over one of the end segments and onto the intermediate segments making the application of the pressure differential superfluous. This may provide the advantage of a simpler assembly process.

Another advantage may be achieved when a tubular sleeve is positioned around the bending section, as the friction between the first area profile and the interior surface of the tubular sleeve is reduced. This further reduces the force required to manipulate the bending section and thus improves the manipulation of the tip part.

In the case where the surface according to the first aspect of the present disclosure forms part of proximal end segment may provide the advantage that adhesion to the proximal end segment may be improved further facilitating assembly with another, separate element of the tip part, for instance a flexible tube or a tubular sleeve.

In the case where the surface according to the first aspect of the present disclosure forms part of distal end segment may provide the advantage that adhesion to the distal end segment may be improved further facilitating assembly with another, separate element of the tip part, for instance a cap or a tubular sleeve.

These advantages may be experienced for bending sections of both polymer and metal materials.

Additionally or alternatively, the first aspect of the present disclosure may relate to a bendable articulated tip part for an endoscope, comprising a tubular sleeve and a bending section having a number of hingedly connected segments including a proximal end segment, a distal end segment, and a plurality of intermediate segments positioned between the proximal end segment and the distal end segment,

wherein at least one segment chosen from the group consisting of the proximal end segment, the distal end segment, and the plurality of intermediate segments includes a surface having a line profile with a one-dimensional, arithmetical mean height parameter, $R_a$, of at least 0.9 micrometres, the line profile being derived from an area profile of the surface as measured by the standard ISO 25178. Additionally, the line profile may be in a longitudinal direction, potentially a proximal-distal direction, of the tip part. Additionally, the one-dimensional, arithmetical mean height parameter, $R_a$, may have an identical range as the two-dimensional, arithmetical mean height parameter, $S_a$.

Additionally or alternatively, the two-dimensional, arithmetical mean height parameter, $S_a$, of the area profile may be at least 0.9, 1.0, 1.1, 1.2, 1.3, 1.4, 1.5, 1.6, 1.7, 1.8, 1.9, 2.0, 2.1, 2.2, or 2.3 micrometres and preferably be measured by the standard ISO 25178.

Additionally, the surface of the at least one segment may be an outer, circumferentially extending surface.

The area profile may be measured using the International Organization for Standardization (ISO) standard "25178: Geometric Product Specifications (GPS) - Surface texture: areal" relating to the analysis of 3D areal surface texture as available on 5 December 2018. The area profile forming the basis of this disclosure should be representative for a substantial area of the bending section, and should not contain surface defects or major discontinuities, for instance the area profile should not be located to span a gap between adjacent segments.

The two-dimensional, arithmetical mean height parameter, $S_a$, of the area profile may be calculated using the following formula in accordance with ISO 25168-3:

$$S_a = \frac{1}{A} \iint_A |z(x,y)| \ \mathrm{d}x\mathrm{d}y$$

wherein A is the surface area of the area profile and |z(x,y)| is the height variation from the mean height plane of the area profile.

A number of line profiles may be derived from the area profile and a one-dimensional, arithmetical, mean height parameter, $R_a$, may be calculated using the following formula for each line profile derived from the area profile and averaging the result in addition to calculating the standard deviation:

$$R_a = \frac{1}{L} \int_L |z(x)| \, dx$$

wherein L is length of the line profile and |z(x)| is the height variation from the mean height plane of the line profile.

The tip part may comprise a cap positioned at a distal end of the tip part. The cap may have a proximal end positioned adjacent to the distal end segment. The cap may have a distal end, potentially forming the distal end of the tip part. The cap may comprise an outer circumferentially extending side wall, potentially enclosing a spacing. The cap may accommodate a camera assembly, potentially positioned in the spacing of the cap. The cap may comprise an end wall positioned at the distal end of the cap. The end wall may comprise a window, which may allow light to propagate there through to an image sensor of the camera assembly. The end wall may comprise an opening, potentially adjacent to the camera assembly, so that a portion of light passing through the opening is received by an image sensor of the camera assembly.

The tip part may comprise a camera assembly positioned at a distal end of the tip part and allowing an operator to inspect a body cavity, when the tip part is inserted into the body cavity. The camera assembly may be positioned in the spacing of the cap. The camera assembly may comprise one or some or all elements selected from the group consisting of: an image sensor configured to capture an image, at least one lens configured to alter light received by the image sensor, a camera housing for supporting the parts of the camera assembly, at least one light source configured to provide illumination for the image sensor, a printed circuit board, at least one signal cable for carrying an image signal from the camera assembly to the operator, and a power cable for supplying the camera assembly with electricity. The printed circuit board may be configured to process a signal from the image sensor. The signal cable and/or the power cable may be connected to printed circuit board. The signal cable may be configured for transmitting an image signal to the operating handle or an output for a monitor. The power cable may be configured to supply power to the printed circuit board.

The tip part or camera assembly may comprise at least one light source(s). The light source(s) may be positioned at a distal end of the tip part potentially so that light emitted from the light source(s) is directed distally. At least one or all of the light source(s) may be light emitting diode(s) and/or light fibre(s). The light source(s) may be configured for providing illumination for the image sensor of the camera assembly. The number of light sources may be at least two or at the most two or exactly two.

The segments may comprise at least one cable passage for accommodating at least one cable, e.g. the signal cable for carrying an image signal and/or the power cable for carrying electricity. The cable passage may comprise a through hole in each of the segments, potentially so as to form a cable passage, that may be extending from the distal end segment through the intermediate segment(s) to the proximal end segment. The cable passage may be positioned adjacent to a centre of the segments. The signal and/or the power cable may be positioned in the cable passage.

The tip part may comprise a working passage. The working passage may be configured for accommodating a tube providing a working channel. The working passage may be different from the cable passage. The working channel may be a suction channel for providing a suction at the distal end of the tip part. The suction channel may be connected to a suction connector, potentially at a handle at the proximal end of the insertion tube. The working channel may allow insertion of surgical instruments there through to the distal end of the tip part. The working passage may be omitted to minimize the size of the tip part.

The tip part may comprise a flexible tube. The flexible tube may be attached to the proximal end segment. The flexible tube may comprise an interior spacing defined by an outer circumferentially extending side wall. The outer circumferentially extending side wall may comprise an inner surface and/or an outer surface. The flexible tube may comprise a distal end, which may be connected to the proximal end segment of the bending section. The flexible tube may comprise a proximal end configured for connection with remaining parts of the endoscope, for instance an operating handle of the endoscope. The flexible tube may be integrally provided in one piece. The flexible tube may comprise or consist essentially of a polymeric material. The flexible tube may surround or enclose the cable passage and/or the working passage and/or the steering wire(s).

The bending section may be a section allowing the tip part to bend relative to non-articulated parts of the insertion tube, for instance the flexible tube or cap, potentially so as to allow an operator to manipulate the tip part, potentially by operating a control element of an operating handle, while inserted into a body cavity of a patient.

Additionally or alternatively, the bending section may be integrally formed, potentially in one piece.

At least one hinge member may interconnect adjacent segments of the bending section with each other, e.g. the proximal end segment with an adjacent intermediate segment and the distal end segment with an adjacent intermediate segment.

Additionally or alternatively, each pair of adjacent segments may be interconnected by at least one, two, or three hinge members. The hinge member(s) may be bridging a gap between adjacent segments. The hinge member may allow adjacent segments to pivot relative to each other to allow the bending section to bend.

Each segment may comprise a proximal surface, potentially except the proximal end segment, facing a distal surface of an adjacent segment forming a gap therein between, and at least one hinge member may bridge the gap. Each segment may comprise a distal surface, potentially except the distal end segment, facing a proximal surface of an adjacent segment forming a gap therein between, and at least one hinge member may bridge the gap. The proximal surface and/or distal surface of each segment may be substantially planar.

Each segment of the bending section may comprise a similar, potentially substantially equal, outer, surface, potentially circumferentially extending around a central, proximal-distal axis of the bending section or tip part. The segments may be substantially cylindrically and/or disc-shaped. The outer surface of each segment may form part of an outer circumferentially extending side wall, which may extend around a central axis, potentially a proximal-distal axis, of the tip part. Each segment may be provided so that the bending section has a uniform outer contour.

Additionally or alternatively, each hingedly interconnected segment may consist essentially of the same material and may be integrally formed, potentially in one piece.

Additionally or alternatively, the hingedly interconnected segments may comprise, or consist essentially of, polypropylene (PP), polyethylene (PE), or polyoxymethylene (POM).

The tip part may comprise at least one, preferably two, steering wire(s). Each steering wire may further be positioned in a steering wire passage of the tip part. Each steering wire passage may be formed by a number of through holes provided in the segments of the tip part. Each steering wire passage may be different from the cable passage and/or the working passage. An end of the steering wire may be secured in the distal end of the tip part, and another end of the steering wire may be connected to a control element, potentially a control lever of the control element. Thus by manipulating the control element or lever the steering wire may be tensioned on one side of the plane of the hinge members, and slacked on the other, thus allowing the bending section to bend in a desired direction.

Additionally, the tip part may comprise a tubular sleeve. The tubular sleeve may at least cover or seal a gap, potentially all gaps, between adjacent segments of the bending section. The tubular sleeve may seal the connection between the bending section and an adjacent element of the tip part, for instance a flexible tube and/or a cap. The tubular sleeve may be attached to the proximal end segment at a proximal end of the tubular sleeve, potentially by a hardened adhesive or laser welding, and/or to the distal end segment at a distal end of the tubular sleeve, potentially by a hardened adhesive or laser welding. The tubular sleeve may provide the tip part and/or the flexible tube with an outer surface configured for insertion into a body cavity, for instance a substantially smooth outer surface and/or an outer surface adapted for being in contact with tissue. The wall thickness of the tubular sleeve may be less than 0.3, 0.25, 0.2, 0.15, 0.1, 0.9, or 0.8 mm.

Additionally or alternatively, the tubular sleeve may be made of a polymeric material, such as polyurethane (PU) or thermoplastic polyurethane (TPU) or a polymer sold under the trademark Pellethane® as of December 2018. The tubular sleeve may be translucent or transparent, potentially to ultraviolet light.

The flexible tube, the cap, the tubular sleeve, and the bending section may be provided separately, and/or as separate components that are attached to each other. The flexible tube and the bending section may be formed as separate parts. The cap and the bending section may be formed as separate parts. The tubular sleeve and the bending section may be formed as separate parts.

In some embodiments, the tip part may be a single-use or disposable tip part, potentially for a single-use or disposable endoscope, and may not be intended for cleaning and/or reusing.

Additionally or alternatively, an outer circumference of the bendable articulated tip part may be substantially uniform along the length of the bending section.

In some embodiments, the two-dimensional, arithmetical mean height parameter, $S_a$, of the area profile may be in the range of 0.9 micrometres to 9.0 micrometres as measured by the standard ISO 25178.

Additionally or alternatively, the one-dimensional, $R_a$, and/or two-dimensional, arithmetical mean height parameter, $S_a$, of the area profile may be in the range of 0.9 micrometres to 9.0, 8.5, 8.0, 7.5, 7.0, 6.5, 6.0, 5.5, 5.0, 4.5, 4.0, 3.5, 3.4, 3.3, 3.2, 3.1, 3.0, 2.9, or 2.8 micrometres.

Additionally or alternatively, the one-dimensional, $R_a$, and/or two-dimensional, arithmetical mean height parameter, $S_a$, of the area profile may be in the range of 1.0 micrometres to 9.0, 8.5, 8.0, 7.5, 7.0, 6.5, 6.0, 5.5, 5.0, 4.5, 4.0, 3.5, 3.4, 3.3, 3.2, 3.1, 3.0, 2.9, or 2.8 micrometres.

Additionally or alternatively, the one-dimensional, $R_a$, and/or two-dimensional, arithmetical mean height parameter, $S_a$, of the area profile may be in the range of 1.1 micrometres to 9.0, 8.5, 8.0, 7.5, 7.0, 6.5, 6.0, 5.5, 5.0, 4.5, 4.0, 3.5, 3.4, 3.3, 3.2, 3.1, 3.0, 2.9, or 2.8 micrometres.

Additionally or alternatively, the one-dimensional, $R_a$, and/or two-dimensional, arithmetical mean height parameter, $S_a$, of the area profile may be in the range of 1.2 micrometres to 9.0, 8.5, 8.0, 7.5, 7.0, 6.5, 6.0, 5.5, 5.0, 4.5, 4.0, 3.5, 3.4, 3.3, 3.2, 3.1, 3.0, 2.9, or 2.8 micrometres.

Additionally or alternatively, the one-dimensional, $R_a$, and/or two-dimensional, arithmetical mean height parameter, $S_a$, of the area profile may be in the range of 1.3 micrometres to 9.0, 8.5, 8.0, 7.5, 7.0, 6.5, 6.0, 5.5, 5.0, 4.5, 4.0, 3.5, 3.4, 3.3, 3.2, 3.1, 3.0, 2.9, or 2.8 micrometres.

Additionally or alternatively, the one-dimensional, $R_a$, and/or two-dimensional, arithmetical mean height parameter, $S_a$, of the area profile may be in the range of 1.4 micrometres to 9.0, 8.5, 8.0, 7.5, 7.0, 6.5, 6.0, 5.5, 5.0, 4.5, 4.0, 3.5, 3.4, 3.3, 3.2, 3.1, 3.0, 2.9, or 2.8 micrometres.

Additionally or alternatively, the one-dimensional, $R_a$, and/or two-dimensional, arithmetical mean height parameter, $S_a$, of the area profile may be in the range of 1.5 micrometres to 9.0, 8.5, 8.0, 7.5, 7.0, 6.5, 6.0, 5.5, 5.0, 4.5, 4.0, 3.5, 3.4, 3.3, 3.2, 3.1, 3.0, 2.9, or 2.8 micrometres.

Additionally or alternatively, the one-dimensional, $R_a$, and/or two-dimensional, arithmetical mean height parameter, $S_a$, of the area profile may be in the range of 1.6 micrometres to 9.0, 8.5, 8.0, 7.5, 7.0, 6.5, 6.0, 5.5, 5.0, 4.5, 4.0, 3.5, 3.4, 3.3, 3.2, 3.1, 3.0, 2.9, or 2.8 micrometres.

Additionally or alternatively, the one-dimensional, $R_a$, and/or two-dimensional, arithmetical mean height parameter, $S_a$, of the area profile may be in the range of 1.7 micrometres to 9.0, 8.5, 8.0, 7.5, 7.0, 6.5, 6.0, 5.5, 5.0, 4.5, 4.0, 3.5, 3.4, 3.3, 3.2, 3.1, 3.0, 2.9, or 2.8 micrometres.

Additionally or alternatively, the one-dimensional, $R_a$, and/or two-dimensional, arithmetical mean height parameter, $S_a$, of the area profile may be in the range of 1.8 micrometres to 9.0, 8.5, 8.0, 7.5, 7.0, 6.5, 6.0, 5.5, 5.0, 4.5, 4.0, 3.5, 3.4, 3.3, 3.2, 3.1, 3.0, 2.9, or 2.8 micrometres.

In some embodiments, the two-dimensional, arithmetical mean height parameter, $S_a$, of the area profile may be in the range of 1.8 micrometres to 4.5 micrometres as measured by the standard ISO 25178.

Additionally or alternatively, the one-dimensional, $R_a$, and/or two-dimensional, arithmetical mean height parameter, $S_a$, of the area profile may be 2.3 $\pm$ 1.0 micrometres or in the range of 1.3 micrometres to 3.4 micrometres as measured by the standard ISO 25178.

In some embodiments, the two-dimensional, arithmetical mean height parameter, $S_a$, of the area profile may be 2340 $\pm$ 520 nanometres or in the range of 1820 nanometres to 2860 nanometres as measured by the standard ISO 25178.

Experiments have shown that a one-dimensional, $R_a$, and/or two-dimensional, arithmetical mean height parameter, $S_a$, of the area profile in this range significantly facilitates fitting of the tubular sleeve on the bending section and make use of a pressure differential, such as vacuum or air blast, superfluous as the tubular sleeve may easily be manually fitted by an operator.

In some embodiments, the surface may be a first, outer surface forming part of at least one of the number of hingedly connected segments, and the area profile may be a first area profile, and the tip part may comprise a tubular sleeve covering at least the intermediate segments of the bending section and may be positioned on the bending section so that the tubular sleeve is in contact with and slides across the first area profile of the first surface during a bending movement of the bending section.

This may improve the manipulation of the tip part in that, during manipulation of the bending section, friction between an inner surface of the tubular sleeve and the first area profile is reduced. This can reduce the force required to manipulate the bending section.

Additionally or alternatively, the first area profile may be located on an outer surface of any one of intermediate segments.

In some embodiments, the area profile may be a second area profile and may be attached by a hardened adhesive to another, separate element of the tip part, for instance a tubular sleeve, a cap, and/or a flexible tube.

This may provide the advantage of improved adhesion between the bending section and the other, separate element.

Additionally or alternatively, the second area profile may be located on an inner or outer surface of the proximal end segment, the distal end segment, or any of the intermediate segments.

Additionally or alternatively, the second area profile may be located on an outer surface of the proximal end segment and a fourth area profile may be located on an outer surface of the distal end segment, a proximal end of the tubular sleeve may be attached to the second area profile by a hardened adhesive and the distal end of the tubular sleeve may be attached to the fourth area profile by a hardened adhesive. This may provide the advantage that any gaps between the segments of the bending section may be radially sealed.

In some embodiments, the area profile may be a second area profile and may be located on the proximal end segment, and wherein the tip part may comprise a flexible tube attached to the second area profile of the bending section by a hardened adhesive.

This may provide the advantage that adhesion between the bending section and the flexible tube is improved.

Additionally or alternatively, the second area profile may be located on an inner or outer surface of the proximal end segment.

In some embodiments, the area profile may be a third area profile and may be located on the distal end segment, and wherein the tip part may comprise a cap attached to the third area profile of the bending section by a hardened adhesive.

This may provide the advantage that adhesion between the bending section and the cap is improved.

In some embodiments, the tip part may further comprise:

a sub-assembly having the bending section and a circumferentially extending, outer flange surface which extends from a first outer circumference of the sub-assembly to a second, smaller, outer circumference of the sub-assembly; and
a tubular sleeve including a rim surface, the tubular sleeve at least covering the intermediate segments of the bending section;
wherein the rim surface of the tubular sleeve faces the flange surface of the sub-assembly.

This may provide a particularly simple arrangement since a flange surface of the sub-assembly facing a rim surface of the tubular sleeve may allow the tubular sleeve to attached to the bending section without being affixed, for instance by an adhesive. Another advantage may be that the assembly of the tip part is simplified in that the tubular sleeve and be slid over the bending section and the flange surface may define a stop for the tubular sleeve.

Additionally or alternatively, an outer surface of the tubular sleeve may be arranged substantially flush with an outer surface of the sub-assembly bounded by the first circumference.

Additionally or alternatively, the sub-assembly may not comprise the tubular sleeve.

Additionally or alternatively, the flange surface may be extending in a substantially tangential direction of the proximal-distal axis or a central axis of the tip part. The flange surface may extend around the entire circumference of the sub-assembly. The flange surface may face an axial direction of the tip part. At least a portion of the flange surface may be substantially axial, for instance by having a normal direction with a component parallel to the proximal-distal axis. Additionally or alternatively, the flange surface may taper off from a first outer circumference of the sub-assembly to a second, smaller, outer circumference of the sub-assembly.

Additionally or alternatively, the flange surface may taper in circumference or diameter potentially from the first circumference to the second circumference. The flange surface may extend from the first outer circumference to the second, smaller, outer circumference by tapering off gradually or continuously. Alternatively, the flange surface may extend discontinuously or abruptly, potentially to form a 90-degree step. The flange surface may taper with an angle, potentially measured in relation to the central, proximal-distal axis, the angle may be equal to or less than 75, 80, 85, or 90 degrees.

Additionally or alternatively, the second circumference may be adjacent to the first circumference potentially separated by the flange surface.

Additionally or alternatively, the first and second circumference may form the boundaries between which the flange surface extends.

Additionally or alternatively, the difference in the diameter of the first and second circumference may be substantially equal to twice the thickness of the tubular sleeve.

Additionally or alternatively, the outer flange surface is a proximal, circumferentially extending, outer flange surface, the proximal flange surface being positioned at or adjacent to the proximal end segment,

wherein the rim surface is a proximal rim surface of the tubular sleeve, and

wherein the sub-assembly further comprises a distal, circumferentially extending, outer flange surface which extends from a third outer circumference of the sub-assembly to a fourth, smaller, outer circumference of the sub-assembly, the distal flange surface being positioned at or adjacent to the distal end segment,

wherein the tubular sleeve comprises a distal rim surface facing the distal, outer flange surface of the sub-assembly.

This may provide the advantage of a tip part with a smaller outer diameter, since the tubular sleeve may be positioned between the proximal and distal flange surfaces and thus does not increase the outer diameter of the tip part. Additionally, a tip part of this type may provide an advantage of aiding in keeping the tubular sleeve between the flange surfaces and thus aiding in preventing the tubular sleeve from sliding off the bending section. Additionally or alternatively, a distal end of the tubular sleeve may be fixed to the bending section proximal to the distal flange surface, and/or a proximal end of the tubular sleeve may be fixed to the bending section distal to the proximal flange surface.

In some embodiments, the tip part may form part of an endoscope.

The term "endoscope" may be defined as a device suitable for examination of natural and/or artificial body openings, e.g. for exploration of a lung cavity. Additionally, or alternatively, the term "endoscope" may be defined as a medical device.

The bendable articulated tip part and/or the bending section and/or the cap and/or the flexible tube may form part of an insertion tube. The insertion tube or a distal end thereof may be suitable for insertion into a body cavity, potentially a lung, through a body opening, potentially a mouth. The body may be a natural and/or artificial body, potentially a human or animal body. The insertion tube may be connected to and extend from an operating handle towards a distal end of the endoscope. The tip part may be positioned at and form the distal end of the insertion tube.

In some embodiments, the endoscope may form part of a system for visually inspecting inaccessible places such as human body cavities, the system may further comprise a monitor, and the endoscope may be connectable to the monitor, and the monitor may allow an operator to view an image captured by a camera assembly of the endoscope.

A second aspect of the present disclosure relates to a mould configured for moulding a bending section for a tip part according to the first aspect of the present disclosure, the mould comprising a mould surface configured for moulding a surface of the bending section and having an area profile with a two-dimensional, arithmetical mean height parameter, $S_a$, of at least 0.9 micrometres.

A third aspect of the present disclosure relates to a method for assembling a tip part for an endoscope, the method comprising the steps of:

- providing a bending section for a tip part according to the first aspect of the present disclosure,
- providing a tubular sleeve, and
- sliding the tubular sleeve over the proximal end segment or distal end segment and onto the intermediate segments during which an inner circumference of the tubular sleeve is in a frictional engagement with an outer surface of the bending section.

An advantage of assembling the tip part in this way may be that the application of a pressure differential to slide the tubular sleeve onto the bending section may be made superfluous. This may provide the advantage of a simpler assembly process.

The step of sliding the tubular sleeve over the proximal end segment or distal end segment and onto the intermediate segments during which an inner circumference of the tubular sleeve is in a frictional engagement with an outer surface of the bending section may be performed without application of a pressure differential, such as an air blast or vacuum.

During the step of sliding the tubular sleeve, substantially the entire inner circumference of the tubular sleeve may be in a frictional engagement with the outer surface of the bending section.

Additionally or alternatively, the method may comprise an additional step of shortening the tubular sleeve potentially by cutting. The step of shortening the tubular sleeve may be performed prior to or after the step of sliding the tubular sleeve. The tubular sleeve may be shortened so that the length of the tubular sleeve at least corresponds to the length of the intermediate segments.

This may provide the advantage of facilitating assembly of the tip part as the tubular sleeve can be slid onto the intermediate segments and any end of the tubular sleeve extending further than the bending section can be cut off. Thus the tubular sleeve does not need to be pre-cut before assembly.

A fourth aspect of the present disclosure relates to a method for obtaining a tip part according to the first aspect of the present disclosure, the method comprising the steps of:

- providing a bending section having a number of hingedly connected segments including a proximal end segment, a distal end segment, and a plurality of intermediate segments positioned between the proximal end segment and the distal end segment, and
- treating a surface of at least one segment chosen from the group consisting of the proximal end segment, the distal end segment, and the plurality of intermediate segments to provide an area profile of the surface with a two-dimensional, arithmetical mean height parameter, $S_a$, of at least 0.9 micrometres.

Additionally or alternatively, the step of treating a first surface of the bending section to provide an area profile of the first surface with an two-dimensional, arithmetical mean height parameter, $S_a$, of at least 0.9 micrometres may be performed by plasma-treating or electrical discharge machining (EDM), which has been shown to significantly increase the adhesion strength of an adhesive adhering to the first surface.

Additionally or alternatively, the step of treating a first surface of the bending section to provide an area profile of the first surface with an two-dimensional, arithmetical mean height parameter, $S_a$, of at least 0.9 micrometres may be performed by sanding and/or grinding.

In some embodiments of the fourth aspect, the method may further comprise:

- providing a mould according to the second aspect of the present disclosure, and
- moulding a bending section for a tip part according to the first aspect of the present disclosure, the bending section having a surface adjacent to the mould surface, thereby providing the bending section and treating the surface of at least one segment chosen from the group consisting of the proximal end segment, the distal end segment, and the plurality of intermediate segments to provide the area profile of the surface with a two-dimensional, arithmetical mean height parameter, $S_a$, of at least 0.9 micrometres.

A person skilled in the art will appreciate that any one or more of the above aspects of the present disclosure and embodiments thereof may be combined with any one or more of the other aspects of the present disclosure and embodiments thereof.

In the following, non-limiting exemplary embodiments will be described in greater detail with reference to the drawings, on which:

Fig. 1a shows a perspective view of an endoscope in which a tip part according to the present disclosure is implemented,
Fig. 1b shows a perspective view of a monitor to which the endoscope of Fig. 1a is connectable,
Fig. 2 shows a perspective view of an embodiment of a tip part according to the present disclosure in which a proximal portion of a flexible tube is omitted,
Fig. 3a shows a side view of the tip part of Fig. 2,
Fig. 3b shows a side view the tip part of Fig. 2 in which a tubular sleeve is omitted,

Fig. 4a shows a side detail view of a proximal end segment of the detail view A of Fig. 3b,
Fig. 4b shows a side detail view of a distal end segment of the detail view B of Fig. 3b,
Fig. 4c shows a side detail view of some intermediate segments of the detail view C of Fig. 3b,

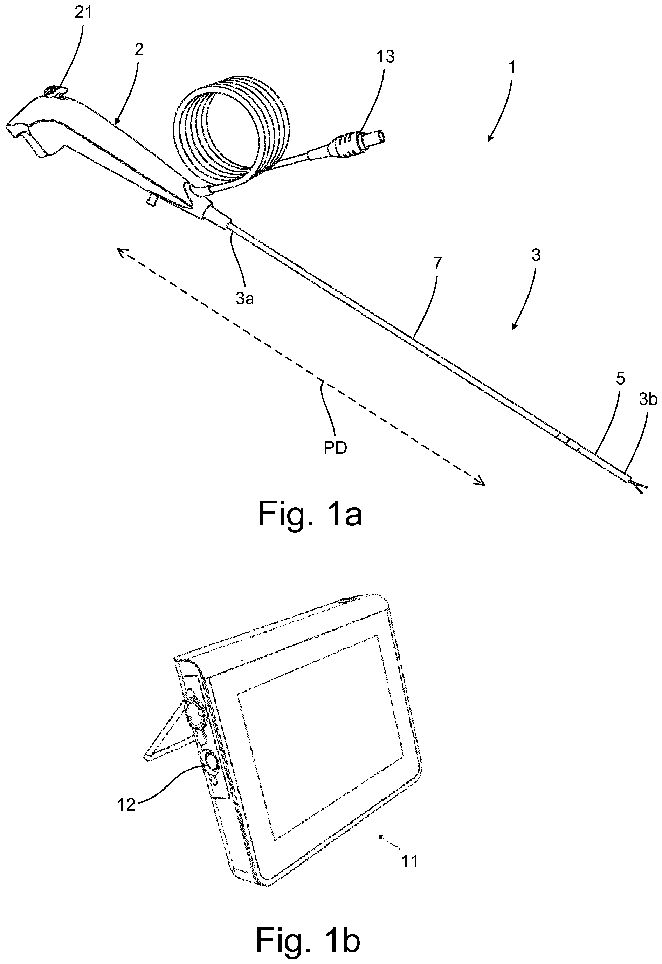

Referring first to Fig. 1a, an endoscope 1 is shown. The endoscope is disposable, and not intended to be cleaned and reused. The endoscope 1 comprises an elongated insertion tube 3. The insertion tube 3 is suitable for insertion into a lung of a body through a mouth. The body can be a natural or artificial body, for instance a human or animal body. At the proximal end 3a of the insertion tube 3 an operating handle 2 is arranged. The operating handle 2 has a control lever 21 for manoeuvring an articulated tip part 5 at the distal end 3b of the insertion tube 3 by means of a steering wire 31a. A camera assembly 61 is positioned in the tip part 5 and is configured to transmit an image signal through a monitor cable 13 of the endoscope 1 to a monitor 13.

In Fig. 1b, a monitor 11 is shown. The monitor 11 may allow an operator to view an image captured by the camera assembly of the endoscope 1. The monitor 11 comprises a cable socket 12 to which a monitor cable 13 of the endoscope 1 can be connected to establish a signal communication between the camera assembly 61 of the endoscope 1 and the monitor 11.

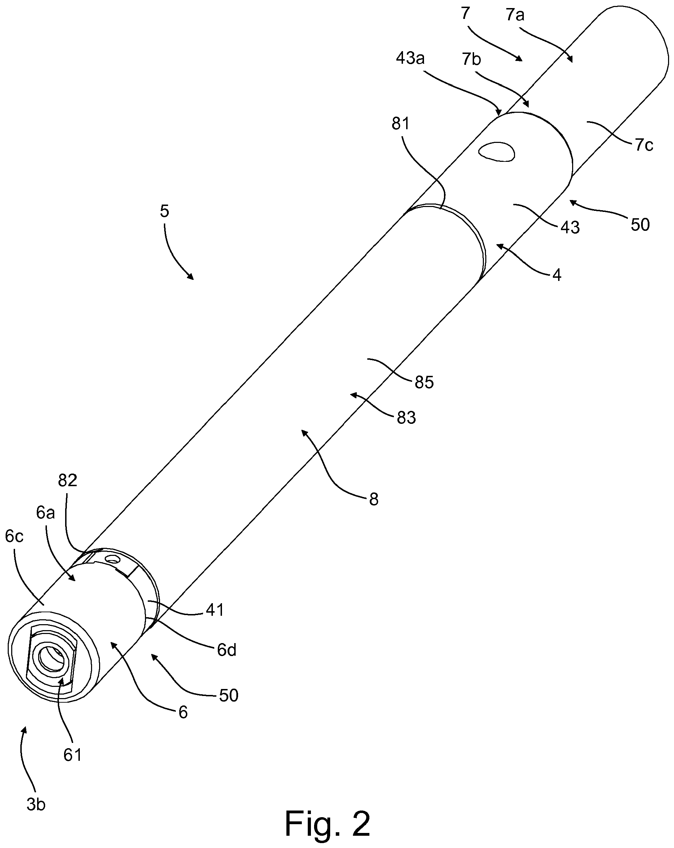

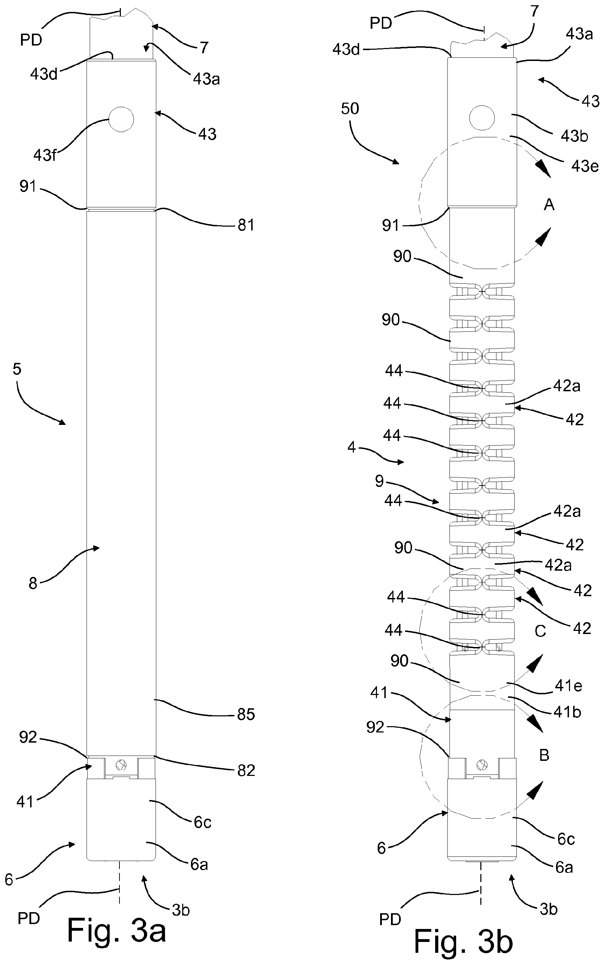

Turning to Fig. 2 and 3a, the distal end 3b of the insertion tube 3 is shown and more specifically the articulated tip part 5. The tip part 5 comprises a sub-assembly 50 including a bending section 4, a cap 6, a camera assembly 6, and a flexible tube 7. A tubular sleeve 8 wraps around the bending section 4 of the sub-assembly 50. The tip part 5 has a substantially uniform outer circumference from the distal end 3b of the tip part 5 to the proximal end 43a of a proximal end segment 43 of the bending section 4. The bending section 4 allows the tip part 5 to bend relative to the flexible tube 7, so as to allow an operator to manipulate the tip part 5 while inserted into a body cavity.

As seen in Figs. 2, 3a, and 3b, the cap 6 includes a circumferentially extending side wall 6a enclosing a spacing accommodating the camera assembly 61. The distal end of cap forms the distal end 3b of insertion tube 3 and the tip part 5. The proximal end 6b of the cap 6 is positioned adjacent and is secured to the distal end segment 41 by a hardened adhesive positioned in a gap in the overlap of the proximal end 6b of the cap 6 and the distal end 41a of the distal end segment 41 as better seen in Fig 5c. The outer surface 85 of the tubular sleeve 8 is arranged substantially flush with the outer surface 6c of the outer circumferentially extending side wall 6a of the cap 6.

As seen in Figs. 2, the camera assembly 61 is positioned at a distal end 3b of the insertion tube 3 and tip part 5 in the spacing of the cap 6 so that an image sensor (not shown) is viewing out through an opening in a distal end wall of the cap 6 to allow an operator to inspect a distal body cavity when the insertion tube 3 is inserted into the body cavity. The camera assembly 61 comprises an image sensor configured to capture an image, at least one lens (not shown) configured to alter light received by the image sensor, a camera housing (not shown) for supporting the parts of the camera assembly 61, two light sources (not shown) configured to provide illumination for the image sensor in the form of light emitting diodes, a printed circuit board (not shown), signal cables (not shown) configured for carrying an image signal from the camera assembly 61 to the operator, and power cables configured for supplying power to the printed circuit board. The signal cables and the power cables are accommodated in a cable tube 32a and are connected to a printed circuit board. The printed circuit board is configured to process a signal from the image sensor and transmit the signal via signal cables to the monitor cable 13 of the handle 2 for output to a monitor 11.

As can be seen in Figs. 2, 3a, and 3b, the flexible tube 7 comprises circumferentially extending, side wall 7a with an outer surface 7c and an inner surface (not shown). The flexible tube 7 comprises a proximal end configured for connection with the operating handle 2 of the endoscope 1 as seen in Fig. 1a. The flexible tube 7 is integrally provided in one piece and consists essentially of a second, polymeric material different from the bending section 4. The flexible tube 7 surrounds and encloses the steering wire 31a and the cable tube 32a with the signal cables and power cables. The flexible tube 7 is positioned in the spacing (not shown) of the proximal end segment 43 by inserting the flexible tube 7 through an opening 43d of the proximal end segment 43 so that the distal end 7b of the flexible tube 7 and the proximal end 43a of the proximal end segment 43 overlaps. The distal end 7b of the flexible tube 7 is secured to the proximal end 43a of the proximal end segment 43 by an adhesive injected into a through hole provided in an outer circumferentially extending side wall 43b of the proximal end segment 43 thereby distributing the adhesive between the side wall 43b and the distal end 7b of the flexible tube 7 and thereafter caused to harden by exposure to ultraviolet light.

As best seen in Figs. 2, and 3a, the tip part 5 further comprises a tubular sleeve 8 including a circumferentially extending, side wall 83 with an outer surface 85 and an inner surface (not shown). The circumferentially extending, side wall 83 of the tubular sleeve 8 extends between a distal rim surface 82 at a distal end and a proximal rim surface 81 at a proximal end. The rim surfaces 81, 82 defines the elongated extent of the tubular sleeve 8. The tubular sleeve 8 wraps around and covers a sleeve surface 90 of the sub-assembly 50 as can be seen on Fig. 3b. The tubular sleeve 8 extends from the proximal end segment 43 to the distal end segment 41 via the intermediate segments 42 and thus covers and seals all gaps between adjacent segments 41, 42, 43 of the bending section 4. The tubular sleeve 8 is attached to the proximal end segment 43 and the distal end segment 41 by a hardened adhesive sealing and adhering the inner surface 84 of the tubular sleeve 8 to the outer surface 43e, 41e of the proximal end segment 43 and the distal end segment 41, respectively, and thereby seals the connections between the bending section 4 and the flexible tube 7 and the cap 6. In an alternative embodiment, the proximal end of the tubular sleeve 8 is attached to the flexible tube 7 and the distal end of the tubular sleeve 8 is attached to the distal end segment 41. The outer surface 85 of the tubular sleeve 8 is arranged substantially flush with the remaining outer surface of the tip part 5. The tubular sleeve 8 is made of transparent thermoplastic polyurethane. Generally, the tubular sleeve 8 may be attached to the bending section 4 or the flexible tube 7 by a hardened adhesive or by laser welding.

Turning to Fig. 3b, the bending section 4 comprises a number of hingedly connected segments including a proximal end segment 43, a distal end segment 41, and a plurality of intermediate segments 42 positioned between the proximal end segment 43 and the distal end segment 41. In the present embodiment, the number of intermediate segments 42 is eleven, but may in principle be less or even greater. The proximal end segment 43 comprises an outer, circumferentially extending, side wall 43b enclosing a spacing (not shown) into which an opening 43d in the proximal end 43a of the proximal end segment 43 provides access. The side wall 43b includes an outer surface 43e. The distal end segment 41 comprises an outer, circumferentially extending side wall 41b enclosing a spacing (not shown) into which an opening (not shown) in the distal end provides access. The side wall 41b includes an outer surface 41e. Each segment 41, 42, 43 is substantially cylindrically shaped and each intermediate segment 42 is cylindrically disc-shaped. Two hinge members 44 of the living hinge type interconnects adjacent segments with each other. The hinge members 44 bridge a gap between adjacent segments. The bending section 4 and each hingedly interconnected segment 41, 42, 43 consist essentially of the same material and are integrally formed in one piece. The material is polypropylene (PP) but may be any suitable material, such as polyethylene (PE) or polyoxymethylene (POM).

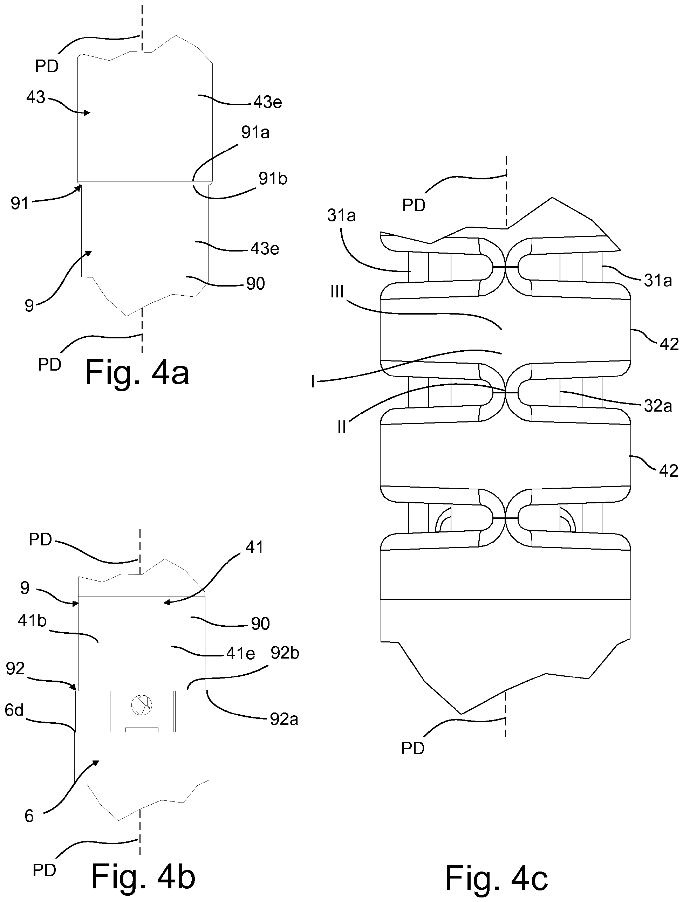

Furthermore, as best seen in Fig. 4a, the sub-assembly 50 includes a proximal, outer flange surface 91 extending around the entire circumference of the proximal end segment 43. The proximal flange surface 91 is positioned at and is integrally formed in one piece by the outer, circumferentially extending surface 43e of the proximal end segment 43.

Furthermore, as best seen in Fig. 4b, the sub-assembly 50 also includes a distal, outer flange surface 92 extending around the entire circumference of the distal end segment 41. The distal flange surface 92 is positioned at and is integrally formed in one piece by the outer, circumferentially extending surface 41e of the distal end segment 41.

As best seen in Fig. 3b, the proximal flange surface 91 and the distal flange surface 92 delimits a recess or cut-out 9 of a smaller circumference relative to the most proximal and distal portions of the bending section 4. The floor of the recess or cut-out 9 is defined by a sleeve surface 90 extending circumferentially around the bending section 4. The circumferentially extending, outer surface 42a of each of the intermediate segments 42 forms part of the sleeve surface 90 along with narrow portions of the end segments 41, 43 adjacent to the flange surfaces 91, 92. The sleeve surface 90 thus has a substantially constant circumference along the proximal-distal axis of the tip part excluding the gaps between adjacent segments 41, 42, 43. The gaps between adjacent segments 41, 42, 43 forms breaks in the sleeve surface 90. An outer circumference of the sleeve surface 90 is substantially narrower than the outer circumference of the remaining parts of the bending section 4 and cap 6 to accommodate the tubular sleeve 8.

Referring now to Figs. 4a, the proximal flange surface 91 extends from a first, proximal, outer circumference 91a of the proximal end segment 43 to a second, smaller, distal, outer circumference 91b of the proximal end segment 43 by gradually tapering off. The flange surface 91 could also extend with other shapes between the circumferences, for instance abruptly or taper linearly off by an angle measured in relation to the central, proximal-distal axis PD or form a step similar to the distal flange surface 92. The proximal flange surface 91 is connected to and directly transitions to the sleeve surface 90 at the second outer circumference 91b.

As best seen in Figs. 4b, the distal flange surface 92 extends abruptly forming a step from a third, proximal, outer circumference 92a of the distal end segment 41 to a fourth, smaller, distal, outer circumference 92b of the distal end segment 41. The flange surface 92 could also extends with other shapes, for instance taper linearly off by an angle measured in relation to the central, proximal-distal axis PD or be rounded. The distal flange surface 92 is connected to and directly transitions to the sleeve surface 90 at the fourth outer circumference 92b. The third circumference 92a could alternatively be provided a proximal rim surface 6d of the cap 6 as the proximal end 6b of the cap 6 could extend further and thus would achieve similar results. As seen in Fig 4b, the distal flange surface 92 does not extend around the entire circumference of the distal end segment 41.

Referring to Figs. 3a, the tubular sleeve 8 is shown positioned in the recess or cut-out 9 formed by the flange surfaces 91, 92 and the sleeve surface 90 and positioned to cover most of the sleeve surface 90. The proximal rim surface 81 of the sleeve 8 is positioned adjacent and distally in relation to the proximal flange surface 91. Additionally, the proximal rim surface 81 faces and is oriented towards the proximal flange surface 91. Further, the proximal rim surface 81 and the proximal flange surface 91 could be positioned to abut but is shown positioned at a distance from each other. The second circumference 91b of the proximal flange surface 91 is substantially equal to the circumference of the inner surface 84 of the sleeve 8 when the tubular sleeve 8 is positioned on the sleeve surface 90. The first circumference 91a of the proximal flange surface 91 is substantially equal to the circumference of the outer surface 85 of the sleeve 8 to ensure a substantially uniform outer circumference along the length of the tip part 5 including the bending section 4, however in practice there might be slight differences. A difference of less than plus/minus 5% may be considered substantially equal.

In this embodiment, the lower bound of the maximum outer circumference or diameter is partially defined by the distal end 7b of the flexible tube 7, thus by positioning the proximal rim surface 81 distally in relation the distal end 7b of the flexible tube 7 it is ensured that the sleeve 8 does not overlap with the distal end 7b of the flexible tube 7. In other embodiments, other parts of the sub-assembly 50 may define the lower bound of the maximum outer circumference or diameter of the tip part 5.

As best seen in Fig. 3a, the distal rim surface 82 of the sleeve 8 is positioned adjacent and proximally in relation to the distal flange surface 92. Additionally, the distal rim surface 82 faces and is oriented towards the distal flange surface 92. Further, the distal rim surface 82 and the distal flange surface 92 could be positioned to abut but is shown positioned at a distance from each other. The fourth circumference 92b of the distal flange surface 92 is substantially equal to the circumference of the inner surface 84 of the sleeve 8. The third circumference 92a of the distal flange surface 92 is substantially equal to the circumference of the outer surface 85 of the sleeve 8 to ensure a substantially uniform outer circumference along the length of the tip part 5 including the bending section 4, however in practice there might be slight differences. A difference of less than plus/minus 5% may be considered substantially equal.

In this embodiment, the lower bound of the maximum outer circumference or diameter is partially defined by the image sensor of the camera assembly 61, thus by positioning the distal rim surface 82 proximally in relation to the image sensor of the camera assembly 61 it is ensured that the sleeve 8 does not overlap with the image sensor. In other embodiments, other parts of the sub-assembly 50 may define the lower bound of the maximum outer circumference or diameter of the tip part 5.

In the present embodiment, the surfaces of the bending section 4 including the sleeve surface 90, the outer surfaces 41e, 42a, 43e of proximal end segment 43, distal end segment 41, and the intermediate segments 42 have been roughened by moulding the bending section in a mould with a roughened mould surface specified to an electrical discharge machining (EDM) finish scale of 29.

In order to confirm the roughness of the surfaces of the bending section, an experiment was set up and carried out in the following way:

two different mould samples each configured for moulding a different type of bending section sample were provided. The mould samples included a mould surface defining the entire surface facing a moulding cavity. The mould samples were manufactured with a roughened mould surface specified to an electrical discharge machining (EDM) finish scale of 29 which in theory approximately corresponds to an $R_a$ value of 2.84 micrometres.

Two types of bending sections were then moulded in the mould cavity of the respective mould sample to obtain two different types of bending section samples with roughened outer surfaces.

An area profile was then obtained at three different locations I, II, III on the mould sample and three corresponding locations I, II, III on each of the bending section samples. In this case, a corresponding location is a location on the bending section sample facing and adjacent to the location on the mould sample during moulding of the bending section sample. The second location II was located on the outer surface of a hinge member adjacent to an intermediate segment 42. The third location III is located on the outer surface of an intermediate segment 42 approximately at the midpoint between two hinge members. The three locations I, II, III are shown in Fig. 4c.

The mould surface of the mould sample and the outer surface of each of the bending section samples were measured using a Sensofar Plu Neox optical confocal microscope which acquires a well-defined set of images obtained by varying the focal depth of a 50x/NA 0.80 objective. The objective had a lateral resolution of approximately 0.3 micrometre and a vertical resolution equal to or less than 3 nanometres. The set of images were converted into a 3D image using SensoScan 3.6 software package.

From the 3D image, three area profiles corresponding to the three locations I, II, III were obtained for the mould sample and for each of the bending section samples. Any unphysical points of the area profiles were levelled and disregarded and the area profiles were then filtered using a Gaussian S filter (1 micrometre) and a L filter (250 micrometres). From these filtered area profiles both a one-dimensional, $R_a$, and a two-dimensional, arithmetical mean height parameter, $S_a$, were calculated using the previously described formula in accordance with ISO 25178. The resulting parameters and a measurement uncertainty in parenthesis for each location and an overall average for all three locations are provided in the following tables. The measurement uncertainty comprises contributions from amplification error, the experimental stitching error, repeatability and reproducibility, and is stated as the standard uncertainty multiplied by the coverage factor, k, of two, which for a normal distribution corresponds to a coverage probability of approximately 95%. The standard uncertainty of measurement is determined in accordance with the EA-4/02 standard. The measurement uncertainty may be used to form a range of confidence, i.e. a number given as X (Y) in any of the below tables may form a range of X $\pm$ Y.

| | Mould type 1 | | Mould type 2 | |
|---|---|---|---|---|
| Location | $S_a$ [nm] | $R_a$ [nm] | $S_a$ [nm] | $R_a$ [nm] |
| I | 1882 | 1650 | 2784 | 2409 |
| II | 1962 | 1860 | 3166 | 2265 |
| III | 2193 | 1927 | 2301 | 1976 |
| Average | 1999 (140) | 1777 (403) | 2686 (364) | 2203 (480) |

Table 1: Average arithmetical mean height parameter results in nano-metres for the mould samples.

| | Sample type 1 | | Sample type 2 | |
|---|---|---|---|---|
| Location | $S_a$ [nm] | $R_a$ [nm] | $S_a$ [nm] | $R_a$ [nm] |
| I | 2353 (131) | 2129 (178) | 2265 (374) | 1849 (172) |
| II | 2199 (150) | 1909 (196) | 2223 (117) | 1887 (172) |
| III | 2500 (291) | 2246 (208) | 2145 (320) | 1868 (177) |
| Average | 2340 (520) | 2100 (1100) | 2200 (640) | 1860 (940) |

Table 2: Average arithmetical mean height parameter results in nano-metres for the bending section samples.

Surprisingly, a tubular sleeve could then be manually slid over the distal end segment and onto the intermediate segments of the bending section samples to arrive at an arrangement as shown in Fig. 3a (omitting the cap and the flexible tube) without conventional use of a pressure differential, such as an air blast or vacuum. This significantly simplified the assembly process as the tubular sleeve did not need to be mounted in a rig in order to apply a pressure differential.

However, the bending section samples and tubular sleeve were assembled using the conventional method and thereafter attached to a cap and a flexible tube by adhesion to arrive at an assembled tip part as shown in Fig. 2 and 3a. A significantly better adhesion was obtained compared to adhesion between a conventional bending section and a cap and a flexible tube.

Afterwards, each assembled tip part was then fitted onto an endoscope to arrive at an arrangement similar to Fig. 1a. It was found that the bending manipulation of these endoscopes having roughened bending sections were improved compared to conventional endoscopes not having a roughened bending sections. It is contemplated that this is due to a reduced friction between the roughened surface of the bending section and the interior surface of the tubular sleeve.

The following is a list of reference numerals used throughout this disclosure. In case of any doubt, the reference numerals of the following list apply.

1        endoscope
11        monitor
12        cable socket
13        monitor cable
2        handle
21        control lever
3        insertion tube
3a        proximal end
3b        distal end
31a        steering wire
32a        cable tube

| | |
|---|---|
| 4 | bending section |
| 41 | distal end segment |
| 41a | distal end |
| 41b | outer circumferentially extending side wall |
| 41d | opening |
| 41e | outer surface |
| 42 | intermediate segment |
| 42a | outer surface |
| 43 | proximal end segment |
| 43a | proximal end |
| 43b | outer circumferentially extending side wall |
| 43d | opening |
| 43e | outer surface |
| 43f | through hole |
| 44 | hinge member |
| 5 | tip part |
| 50 | sub-assembly |
| 6 | cap |
| 6a | circumferentially extending side wall |
| 6b | proximal end |
| 6c | outer surface |
| 6d | proximal rim surface |
| 61 | camera assembly |
| 7 | flexible tube |
| 7a | circumferentially extending side wall |
| 7c | outer surface |
| 8 | tubular sleeve |
| 81 | proximal rim surface |
| 82 | distal rim surface |
| 83 | circumferentially extending side wall |
| 85 | outer surface |
| 9 | recess or cut-out |
| 90 | sleeve surface |
| 91 | proximal flange surface |
| 91a | first circumference |
| 91b | second circumference |
| 92 | distal flange surface |
| 92a | third circumference |
| 92b | fourth circumference |
| PD | proximal-distal axis |
| I | first location |
| II | second location |
| III | third location |

**Claims**

1.  A bendable articulated tip part for an endoscope, comprising a bending section having a number of hingedly connected segments including a proximal end segment, a distal end segment, and a plurality of intermediate segments positioned between the proximal end segment and the distal end segment,
    wherein at least one segment chosen from the group consisting of the proximal end segment, the distal end segment, and the plurality of intermediate segments includes a surface having an area profile with a two-dimensional, arithmetical mean height parameter, $S_a$, of at least 0.9 micrometres as measured by the standard ISO 25178.

2.  A bendable articulated tip part according to claim 1, wherein the two-dimensional, arithmetical mean height parameter, $S_a$, of the area profile is in the range of 0.9 micrometres to 9.0 micrometres as measured by the standard ISO 25178.

3.  A bendable articulated tip part according to any of the previous claims, wherein the two-dimensional, arithmetical mean height parameter, $S_a$, of the area profile is in the range of 1.8 micrometres to 4.5 micrometres as measured by the standard ISO 25178.

4.  A bendable articulated tip part according to any of the previous claims, wherein the two-dimensional, arithmetical mean height parameter, $S_a$, of the area profile is 2340 $\pm$ 520 nanometres or in the range of 1820 nanometres to 2860 nanometres as measured by the standard ISO 25178.

5.  A bendable articulated tip part according to any of the previous claims, wherein the surface is a first, outer surface forming part of at least one of the number of hingedly connected segments, and the area profile is a first area profile, and the tip part comprises a tubular sleeve covering at least the intermediate segments of the bending section and being positioned on the bending section so that the tubular sleeve is in contact with and slides across the first area profile of the first surface during a bending movement of the bending section.

6.  A bendable articulated tip part according to any of the previous claims, wherein the area profile is a second area profile and is attached by a hardened adhesive to another, separate element of the tip part, for instance a tubular sleeve, a cap, and/or a flexible tube.

7.  A bendable articulated tip part according to any of the previous claims, wherein the area profile is a second area profile and is located on the proximal end segment, and wherein the tip part comprises a flexible tube attached to the second area profile of the bending section by a hardened adhesive.

8.  A bendable articulated tip part according to any of the previous claims, wherein the area profile is a third area profile and is located on the distal end segment, and wherein the tip part comprises a cap attached to the third area profile of the bending section by a hardened adhesive.

9.  A bendable articulated tip part according to any of the previous claims, further comprising:

    a sub-assembly having the bending section and a circumferentially extending, outer flange surface which extends from a first outer circumference of the sub-assembly to a second, smaller, outer circumference of the sub-assembly; and
    a tubular sleeve including a rim surface, the tubular sleeve at least covering the intermediate segments of the bending section;
    wherein the rim surface of the tubular sleeve faces the flange surface of the sub-assembly.

10. An endoscope comprising a tip part according to any of the previous claims.

11. A system for visually inspecting inaccessible places such as human body cavities, the system comprising:
    an endoscope according to claim 10, and a monitor, wherein the endoscope is connectable to the monitor, and the monitor allow an operator to view an image captured by a camera assembly of the endoscope.

12. A mould configured for moulding a bending section for a tippart according to any one of the claims 1 to 9, the mould comprising a mould surface configured for moulding a surface of the bending section and having an area profile with a two-dimensional, arithmetical mean height parameter, $S_a$, of at least 0.9 micrometres.

13. A method for assembling a tip part for an endoscope, the method comprising the steps of:

    - providing a bending section for a tip part according to any one of the claims 1 to 9,
    - providing a tubular sleeve, and
    - sliding the tubular sleeve over the proximal end segment or distal end segment and onto the intermediate segments during which an inner circumference of the tubular sleeve is in a frictional engagement with an outer surface of the bending section.

14. A method for obtaining a tip part according to claims 1 to 9, the method comprising the steps of:

    - providing a bending section having a number of hingedly connected segments including a proximal end segment, a distal end segment, and a plurality of intermediate segments positioned between the proximal end segment and the distal end segment, and

- treating a surface of at least one segment chosen from the group consisting of the proximal end segment, the distal end segment, and the plurality of intermediate segments to provide an area profile of the surface with a two-dimensional, arithmetical mean height parameter, $S_a$, of at least 0.9 micrometres.

15. A method according to claim 14, wherein the method further comprises:

- providing a mould according to claim 12, and
- moulding a bending section for a tip part according to any one of the claims 1 to 9, the bending section having a surface adjacent to the mould surface, thereby providing the bending section and treating the surface of at least one segment chosen from the group consisting of the proximal end segment, the distal end segment, and the plurality of intermediate segments to provide the area profile of the surface with a two-dimensional, arithmetical mean height parameter, $S_a$, of at least 0.9 micrometres.

Fig. 1a

Fig. 1b

Fig. 2

Fig. 3a

Fig. 3b

Fig. 4a

Fig. 4b

Fig. 4c

Europäisches
Patentamt

European
Patent Office

Office européen
des brevets

## EUROPEAN SEARCH REPORT

Application Number

EP 19 16 0528

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/262180 A1 (DANITZ DAVID J [US] ET AL) 14 October 2010 (2010-10-14) | 1,5, 9-11,13, 14 | INV.<br>A61B1/00<br>A61B1/005 |
| Y | * paragraphs [0013], [0061], [0062], [0089], [0092], [0095], [0099]; figure 18 * | 2-4,6-8, 12,15 | A61M25/00<br>A61M25/01 |
| Y | JP 2002 224019 A (OLYMPUS OPTICAL CO) 13 August 2002 (2002-08-13)<br>* abstract; figures 2,7 * | 2-4,12, 15 | |
| Y | US 2017/028684 A1 (IMAI SHIGERU [JP] ET AL) 2 February 2017 (2017-02-02)<br>* paragraph [0043] * | 6-8 | |
| A | JP H06 189898 A (OLYMPUS OPTICAL CO) 12 July 1994 (1994-07-12)<br>* abstract; figures 1,2 * | 1-15 | |

| | |
|---|---|
| | TECHNICAL FIELDS SEARCHED (IPC) |
| | A61B<br>A61M |

The present search report has been drawn up for all claims

| Place of search | Date of completion of the search | Examiner |
|---|---|---|
| Munich | 22 August 2019 | Rick, Kai |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

.............................................................................................

& : member of the same patent family, corresponding
  document

EPO FORM 1503 03.82 (P04C01)

## ANNEX TO THE EUROPEAN SEARCH REPORT
## ON EUROPEAN PATENT APPLICATION NO.

EP 19 16 0528

This annex lists the patent family members relating to the patent documents cited in the above-mentioned European search report.
The members are as contained in the European Patent Office EDP file on
The European Patent Office is in no way liable for these particulars which are merely given for the purpose of information.

22-08-2019

| Patent document cited in search report | Publication date | Patent family member(s) | Publication date |
|---|---|---|---|
| US 2010262180 A1 | 14-10-2010 | AU 2005309974 A1 | 01-06-2006 |
| | | CA 2588286 A1 | 01-06-2006 |
| | | CA 2850651 A1 | 01-06-2006 |
| | | CN 101106951 A | 16-01-2008 |
| | | EP 1833398 A2 | 19-09-2007 |
| | | EP 2823772 A1 | 14-01-2015 |
| | | EP 3391804 A2 | 24-10-2018 |
| | | JP 5188811 B2 | 24-04-2013 |
| | | JP 5409731 B2 | 05-02-2014 |
| | | JP 5530991 B2 | 25-06-2014 |
| | | JP 2008521485 A | 26-06-2008 |
| | | JP 2012005868 A | 12-01-2012 |
| | | JP 2012005869 A | 12-01-2012 |
| | | US 2005107667 A1 | 19-05-2005 |
| | | US 2008262538 A1 | 23-10-2008 |
| | | US 2010261964 A1 | 14-10-2010 |
| | | US 2010261971 A1 | 14-10-2010 |
| | | US 2010262075 A1 | 14-10-2010 |
| | | US 2010262161 A1 | 14-10-2010 |
| | | US 2010262180 A1 | 14-10-2010 |
| | | US 2013340559 A1 | 26-12-2013 |
| | | US 2017095305 A1 | 06-04-2017 |
| | | WO 2006057702 A2 | 01-06-2006 |
| JP 2002224019 A | 13-08-2002 | JP 3602803 B2 | 15-12-2004 |
| | | JP 2002224019 A | 13-08-2002 |
| US 2017028684 A1 | 02-02-2017 | CN 106029370 A | 12-10-2016 |
| | | EP 3192653 A1 | 19-07-2017 |
| | | JP 5945650 B1 | 05-07-2016 |
| | | JP WO2016038945 A1 | 27-04-2017 |
| | | US 2017028684 A1 | 02-02-2017 |
| | | WO 2016038945 A1 | 17-03-2016 |
| JP H06189898 A | 12-07-1994 | NONE | |

EPO FORM P0459

For more details about this annex : see Official Journal of the European Patent Office, No. 12/82